# EUROPEAN PATENT APPLICATION

(11) **EP 4 252 655 A1**
(43) Date of publication of application: **04.10.2023**
(21) Application number: 21897772.6
(22) Date of filing: 15.11.2021
(51) Int. Cl.: A61B 6/00, A61B 5/00

(54) **MEDICAL IMAGE DISPLAY SYSTEM, MEDICAL IMAGE DISPLAY METHOD, AND PROGRAM**

(30) Priority: 25.11.2020 JP 2020195467
(71) Applicant: Panasonic Holdings Corporation, Osaka 571-8501 (JP)
(72) Inventor: NOTOSHI, Tomoharu, Osaka 571-0057 (JP); ISHII, Masahiro, Osaka 571-0057 (JP); KONDO, Kenji, Osaka 571-0057 (JP); KIMURA, Hirohiko, Fukui 910-1193 (JP); SAKAI, Toyohiko, Fukui 910-1193 (JP); TANAKA, Masato, Fukui 910-1193 (JP); FUJIMOTO, Shinichi, Fukui 910-1193 (JP)
(74) Representative: Novagraaf International SA
(86) International application number: PCT/JP2021/041894
(87) International publication number: WO 2022/113798

(57) **Abstract**

A medical image display system displays a medical image including: a first display area for displaying a processed image (54a) in which at least one annotation indicating a result of detection of at least one anatomical structure shown in a captured image (32b) of a subject is superimposed on the captured image (32b); and a second display area for displaying at least one structure label for identifying the at least one anatomical structure. The medical image display system includes: a display (54) that displays the medical image; and a controller (53) that changes, in response to an input received for one of (i) the at least one annotation and (ii) the at least one structure label from a user, a display form of the other of (i) the at least one annotation and (ii) the at least one structure label.

## Description

### [Technical Field]

The present invention relates to a medical image display system, a medical image display method, and a program.

### [Background Art]

In the medical field, digitization of captured medical images has proceeded. For example, a study has been conducted on image processing performed on a captured image so as to identify predetermined pathology. Meanwhile, it has been suggested that, in addition to a processed image obtained as a result of the image processing, a comparison image (a captured image before the image processing, for example) be used for comparison with this processed image at the time of diagnosis. In response to this, Patent Literature (PTL) 1 discloses an information processing device that outputs a processed image and a comparison image.

### [Citation List]

### [Patent Literature]

[PTL 1] Japanese Unexamined Patent Application Publication No. 2015-100424

### [Summary of Invention]

### [Technical Problem]

For a diagnosis made using the processed image, prevention of a decrease in the diagnostic efficiency is desirable.

In view of this, the present disclosure provides a medical image display system and a medical image display method that are capable of preventing a decrease in the diagnostic efficiency.

### [Solution to Problem]

In accordance with an aspect of the present disclosure, a medical image display system displays a medical image including: a first display area for displaying a processed image in which at least one annotation indicating a result of detection of at least one anatomical structure shown in a captured image of a subject is superimposed on the captured image; and a second display area for displaying at least one structure label for identifying the at least one anatomical structure. The medical image display system includes: a display that displays the medical image; and a controller that changes, in response to an input received for one of (i) the at least one annotation and (ii) the at least one structure label from a user, a display form of the other of (i) the at least one annotation and (ii) the at least one structure label.

In accordance with another aspect of the present disclosure, a medical image display method is a method of displaying a medical image including: a first display area for displaying a processed image in which at least one annotation indicating a result of detection of at least one anatomical structure shown in a captured image of a subject is superimposed on the captured image; and a second display area for displaying at least one structure label for identifying the at least one anatomical structure. The medical image display method includes changing, in response to an input received for one of (i) the at least one annotation and (ii) the at least one structure label from a user, a display form of the other of (i) the at least one annotation and (ii) the at least one structure label.

In accordance with still another aspect of the present disclosure, a program causes a computer to execute the above-described medical image display method.

### [Advantageous Effects of Invention]

The present disclosure provides a medical image display system, a medical image display method, and a program that are capable of preventing a decrease in the diagnostic efficiency.

### [Brief Description of Drawings]

[FIG. 1] FIG. 1 is a block diagram illustrating a configuration of a diagnostic support system according to Embodiment 1.
[FIG. 2A] FIG. 2A illustrates an example of an anatomical structure.
[FIG. 2B]

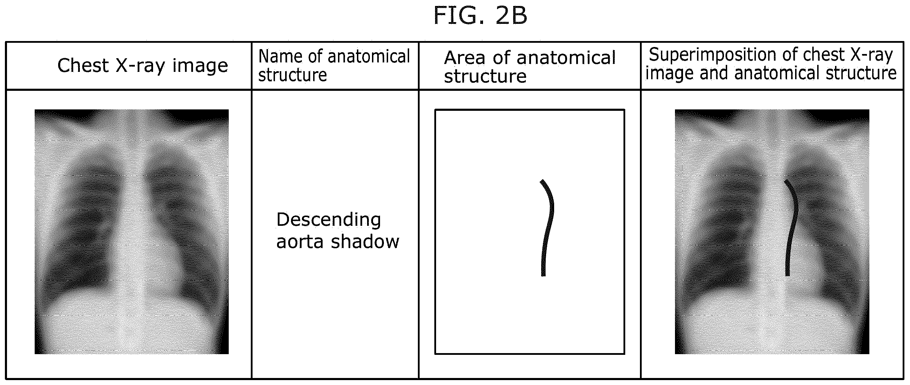

   FIG. 2B illustrates another example of an anatomical structure.
[FIG. 2C]

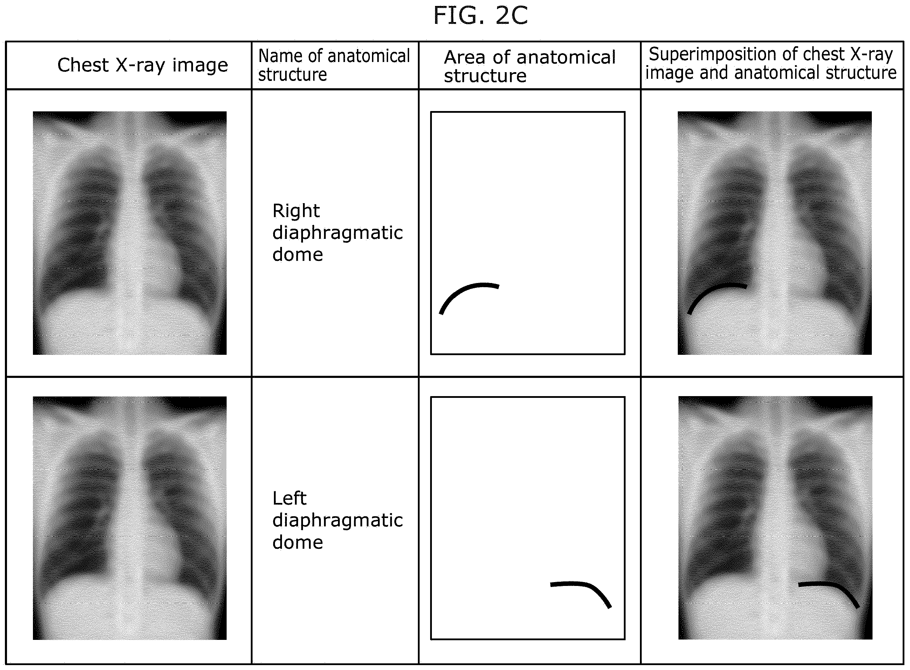

   FIG. 2C illustrates another example of an anatomical structure.
[FIG. 2D]

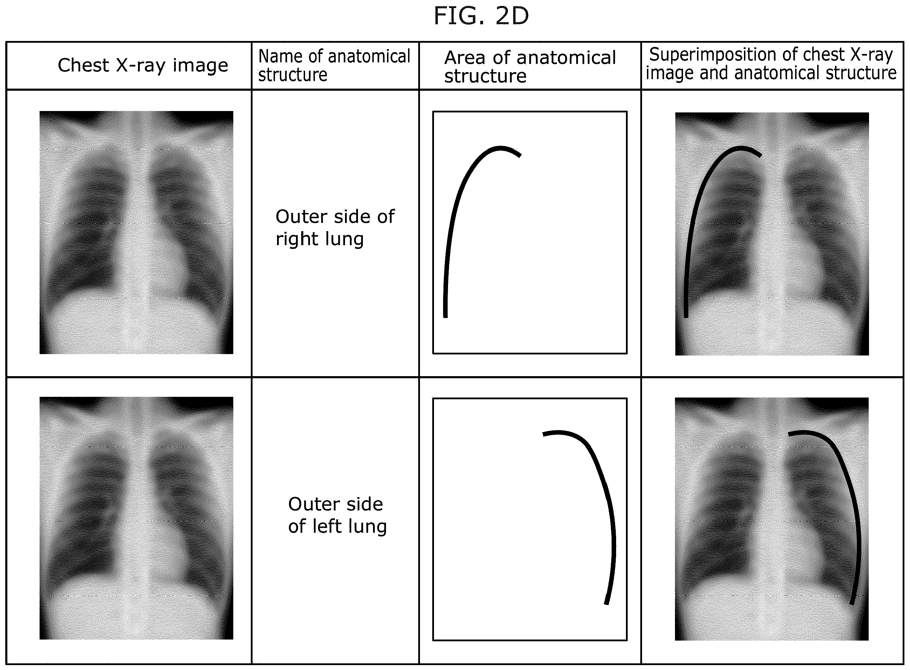

   FIG. 2D illustrates another example of an anatomical structure.
[FIG. 3]

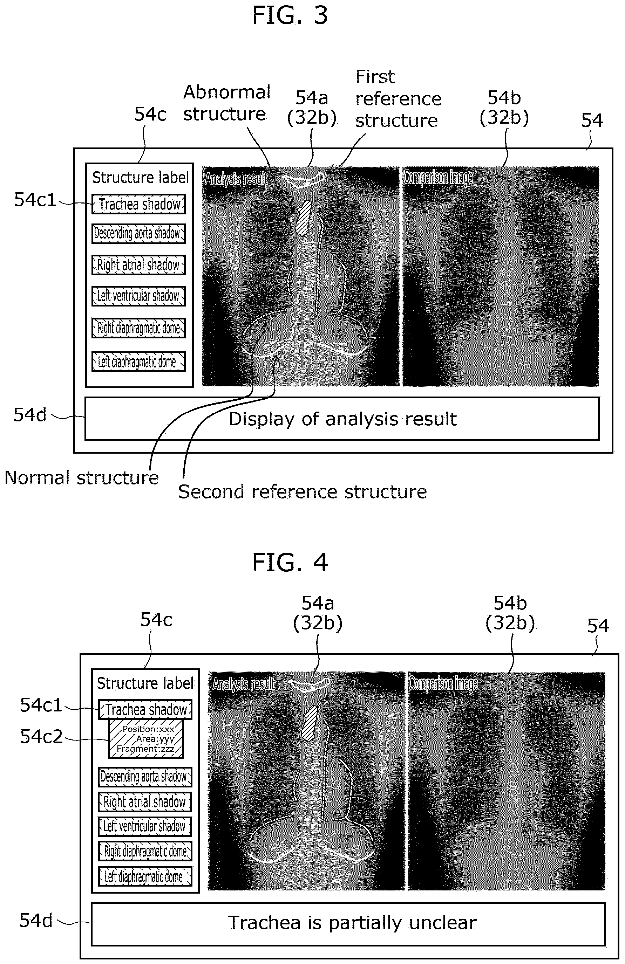

   FIG. 3 illustrates an example of a medical image displayed by a display according to Embodiment 1.
[FIG. 4]
   FIG. 4 illustrates an example of a medical image shown when the trachea shadow is selected among segmentations.
[FIG. 5]

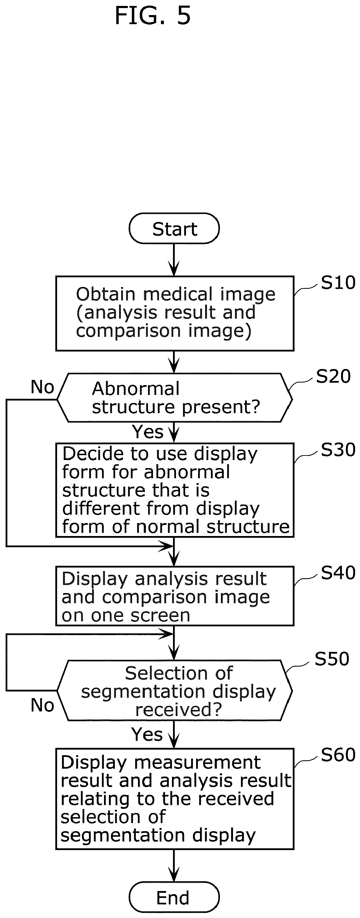

   FIG. 5 is a flowchart of a main operation performed by a viewer device according to Embodiment 1.
[FIG. 6]

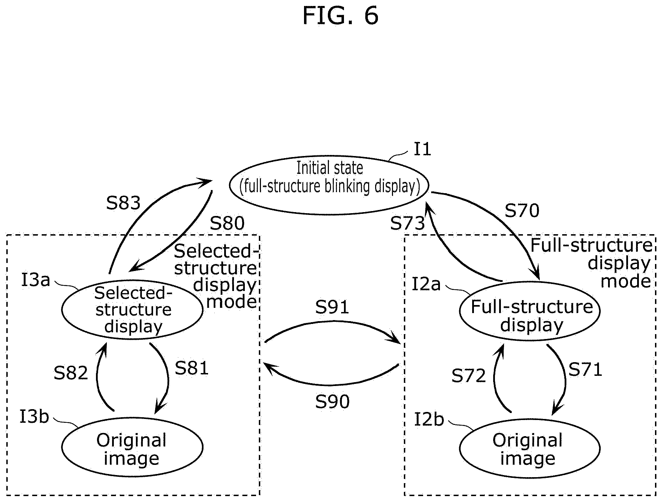

   FIG. 6 is a state transition diagram illustrating transitions of a display state of the viewer device according to Embodiment 1.
[FIG. 7]

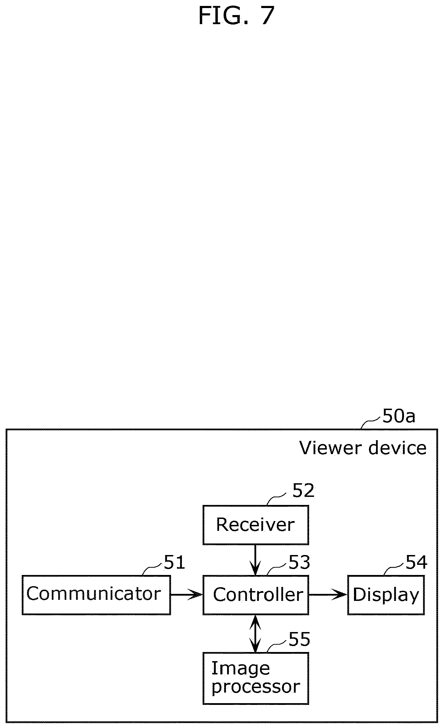

   FIG. 7 is a block diagram illustrating a configuration of a viewer device according to Embodiment 2.
[FIG. 8A]

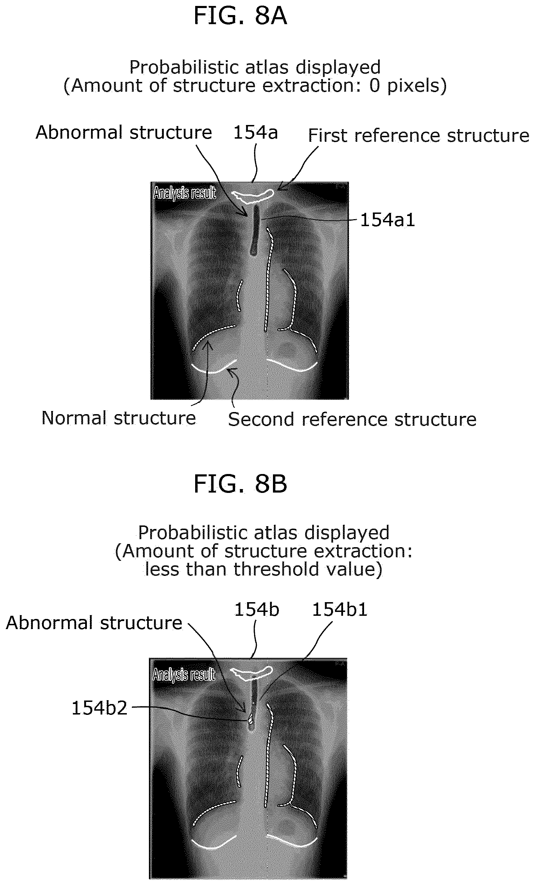

   FIG. 8A illustrates a first example of a medical image displayed by a display according to Embodiment 2.
[FIG. 8B]
   FIG. 8B illustrates a second example of a medical image displayed by the display according to Embodiment 2.
[FIG. 8C]

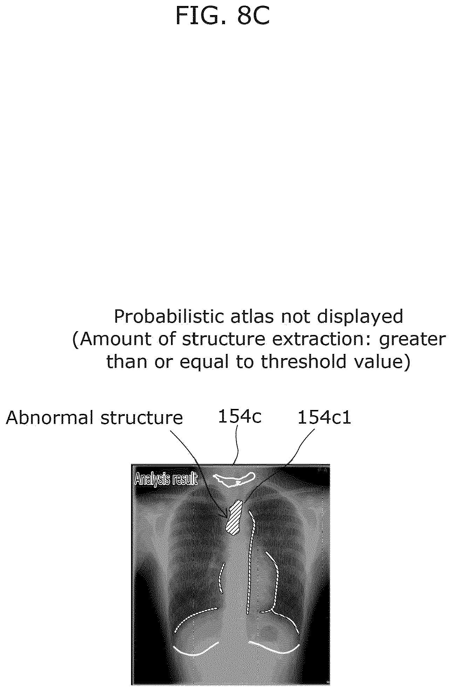

   FIG. 8C illustrates a third example of a medical image displayed by the display according to Embodiment 2.
[FIG. 9]

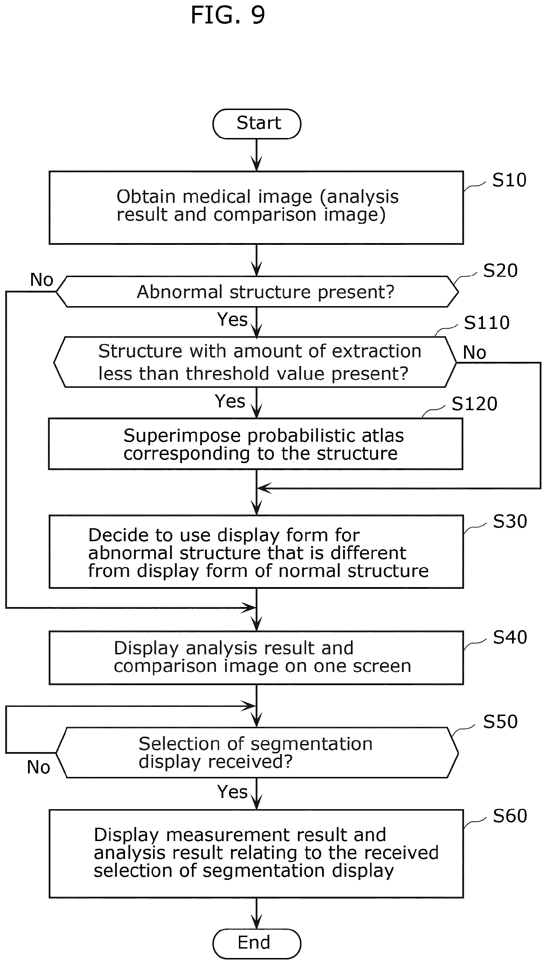

   FIG. 9 is a flowchart of a main operation performed by the viewer device according to Embodiment 2.

### [Description of Embodiments]

### (Underlying Knowledge Forming Basis of the Present Disclosure)

Recent years have seen the development of computer-aided diagnosis (CAD) using artificial intelligence (AI) technology that assists medical doctors in making diagnoses. CAD is achieved by a computer that quantifies and analyzes a captured medical image. A result of the quantization and analysis is used as a second opinion by, for example, a medical doctor (a user) for diagnostic imaging. The output of the processed image and the comparison image as described in Background Art is an example of assistance to the user in making a diagnosis.

However, CAD is not yet widespread on the market and there are various AI types (for X-ray images and ultrasonic images, for example). Therefore, from the view of preventing a decrease of diagnostic efficiency, it has been desired that the user can easily get to know what kind of functions an AI technology used by the user has (for example, which abnormality the AI can detect).

In response to this, the present inventors have earnestly examined a medical image display system, a medical image display method, and a program that are capable of preventing a decrease in the diagnostic efficiency of user's diagnose, even if the user uses a diagnosis support technology, such as a CAD, which is not widely recognized. As a result, the present inventors have invented a medical image display system, a medical image display method, and a program that are described below. Note that a medical image includes a medical image of a user captured by an imaging device, and is used for a diagnosis to be made by a user.

Hereinafter, embodiments of the present disclosure will be described in detail with reference to the accompanying Drawings. The following embodiments are specific examples of the present disclosure. The numerical values, shapes, materials, elements, arrangement and connection configuration of the elements, steps, the order of the steps, etc., described in the following embodiments are merely examples, and are not intended to limit the present disclosure. Note that the respective figures are schematic diagrams and are not necessarily precise illustrations. Additionally, components that are essentially the same share like reference signs in the figures. Accordingly, overlapping explanations thereof are omitted or simplified.

It should also be noted that the following description may include numerical value ranges and expressions using "constant," "the same," or the like to indicate relationships between the constituent elements. However, such numerical value ranges and expressions do not mean exact meanings only. They also mean the substantially same ranges including a difference of, for example, about several % from the completely same range.

### [Embodiment 1]

### [1-1. Configuration of Diagnostic Support System]

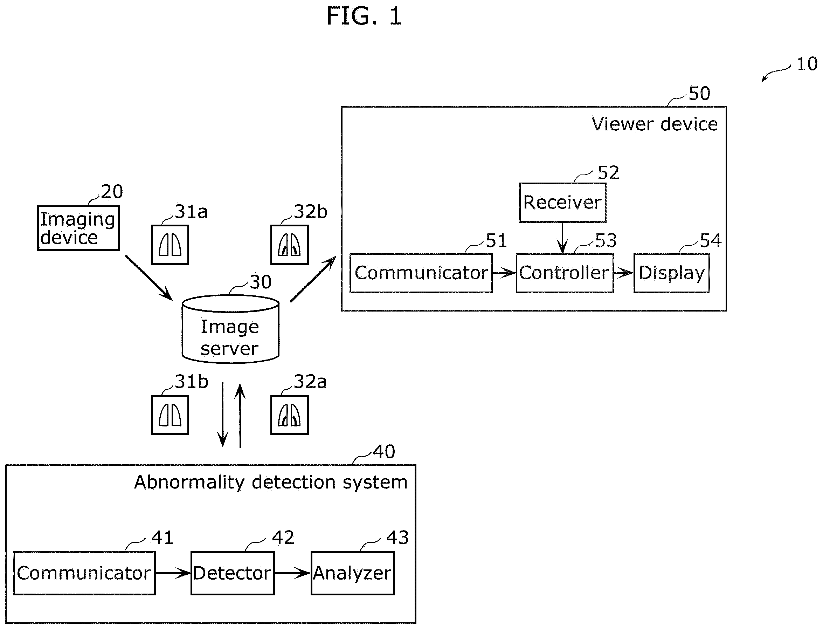

FIG. 1 is a block diagram illustrating a configuration of diagnostic support system 10 according to the present embodiment. Diagnostic support system 10 assists, for example, a medical doctor to efficiently make a diagnosis on the basis of a medical image. Examples of the diagnosis include, but are not limited to, a diagnosis of an anatomical structure.

As illustrated in FIG. 1, diagnostic support system 10 includes imaging device 20, image server 30, abnormality detection system 40, and viewer device 50. Imaging device 20, image server 30, abnormality detection system 40, and viewer device 50 are communicatively connected to one another via a communication channel, such as the Internet.

Imaging device 20 captures an image of a human anatomical structure to generate captured image 31a. In the present embodiment, imaging device 20 is an X-ray imaging device that generates a chest X-ray image as a captured medical image and then stores the captured medical image into image server 30. The captured medical image is not limited to a chest X-ray image and may be an X-ray image of a different section. The captured medical image may also be an image provided by computed tomography (CT) scan, positron emission tomography (PET)/CT scan, single photon emission tomography (SPECT) scan, magnetic resonance imaging (MRI), ultrasound, X-ray, mammography, angiography, fluorography, or photomicrography, or an image provided by any combination of these.

Imaging server 30 is a data server that holds captured image 31a generated by imaging device 20 and captured image 32a generated by abnormality detection system 40 after detection and that provides viewer device 50 with captured image 32b held. For example, imaging server 30 is a computer device including a storage, such as a hard disk. Note that data exchanged between imaging server 30 and a different device includes not only a captured image but also various kinds of information associated with the captured image (such as imaging date and time, information about a patient, a result of detection of an anatomical structure, and a result of analysis). Moreover, the data may further include information about a function of abnormality detection system 40. The information about the function of abnormality detection system 40 includes information indicating an anatomical structure on which abnormality detection can be performed by abnormality detection system 40. If detector 42 uses a learned machine learning model, the information about the function of abnormality detection system 40 includes information indicating an anatomical structure on which abnormality detection can be performed by the learned machine learning model. Here, the patient is an example of a subject to be imaged by imaging device 20.

On the basis of captured image 31b that is a detection target obtained from image server 30, abnormality detection system 40 detects the presence or absence of a medical abnormality in a pre-specified anatomical structure included in captured image 31b. Abnormality detection system 40 includes communicator 41, detector 42, and analyzer 43.

Communicator 41 obtains captured image 31b from image server 30 through communication. Communicator 41 includes a communication circuit, for example. Communicator 41 is an example of an obtainer.

Detector 42 detects an anatomical structure from captured image 31b that is a detection target obtained from image server 30. Detector 42 performs this detection by extracting at least one preset anatomical structure from captured image 31a, for example. Detector 42 detects a shadow of at least one preset organ or the like from captured image 31b, for example. Detector 42 outputs, by superimposing on captured image 31b, information indicating an anatomical structure, for example. The information indicating the anatomical structure is an example of an annotation. The annotation used here refers to information added to (or associated with) the image and indicating a result of detection of the anatomical structure by detector 42, for example. This information may include information about the size, shape, and location of the anatomical structure, for example. The annotation may also include data indicating an area of the anatomical structure and an output value of a segmentation network (such as reliability for each pixel). The data indicating the area of the anatomical structure may be binary data or multivalued data.

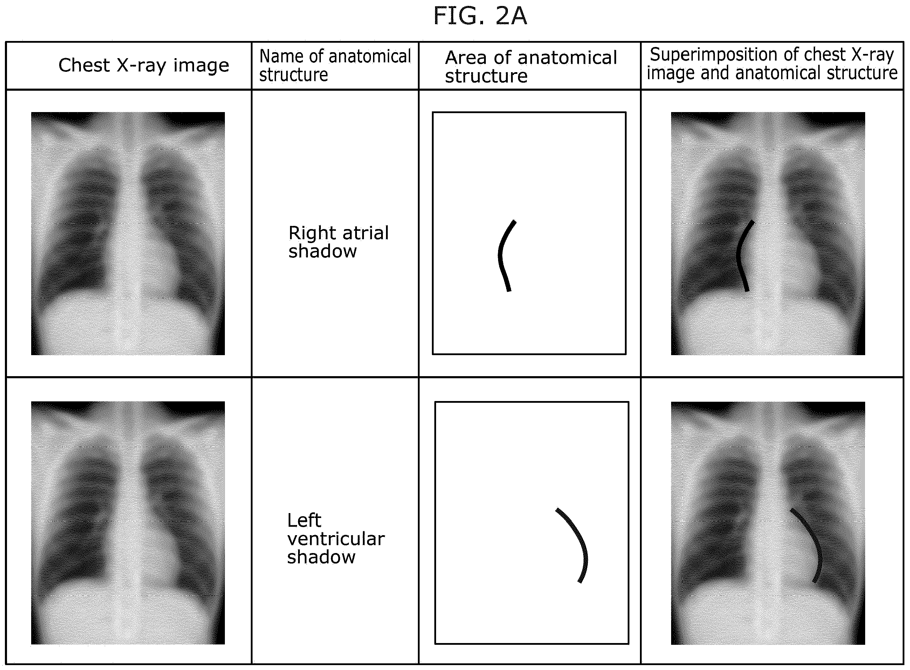

In the present embodiment, detector 42 extracts an anatomical structure using a learned model (such as a neural network model) that performs segmentation to differentiate among anatomical structures, for example. The learned model has previously learned to detect anatomical structures. For example, the learned model, which is stored in a storage device (not shown), uses a captured image of a normal anatomical structure as input data and learns through machine-learning that information indicating the area of the normal anatomical structure in the captured image is correct data. The area of the normal anatomical structure is shown in the column of "Area of anatomical structure" in FIG. 2A to FIG. 2D.

In this case, a result of the detection (output) by detector 42 is captured image 31b on which a segmentation display (segmentation) is superimposed. The following describes, as an example, that the detection result is captured image 31b on which the segmentation display is superimposed. Here, the segmentation display shows a result of extracting an anatomical structure, and is information indicating a contour or area of the anatomical structure. The segmentation display is an example of the annotation.

As described above, the learned model has learned using the captured image of the normal anatomical structure. Thus, if an abnormal anatomical structure is present, detector 42 fails to detect this abnormal anatomical structure. By taking advantage of this, abnormality detection system 40 enables wide detection by which abnormality detection is not limited to finding a specific disease. Note that the learned model may have previously learned to detect the presence or absence of a specific disease, for example.

Analyzer 43 analyzes the result of the detection performed by detector 42. For example, analyzer 43 makes this analysis on the basis of at least one of the size, shape, or location of the segmentation display of captured image 31b outputted from detector 42. On the basis of captured image 31b outputted from detector 42, analyzer 43 determines whether the anatomical structure has an abnormality and generates information (an example of a result of the analysis) indicating a reason for determining that the abnormality is present.

More specifically, communicator 41, detector 42, and analyzer 43 included in abnormality detection system 40 are implemented by at least one computer that includes: a nonvolatile memory that holds a program; a volatile memory as a temporary working space; a processor that executes the program; and an input-output circuit that includes a communication interface and a communication port.

Furthermore, communicator 41, detector 42, and analyzer 43 included in abnormality detection system 40 may be implemented by a single computer or image processing device, or may be implemented separately by a plurality of computers or image processing devices that are connected via a communication channel.

Note that abnormality detection system 40 need not include analyzer 43.

Viewer device 50 displays a medical image including a captured image. In response to an instruction received from the user via receiver 52, viewer device 50 displays captured image 32b stored in image server 30 and various kinds of information associated with captured image 32b, in various display forms. Viewer device 50 includes communicator 51, receiver 52, controller 53, and display 54. Viewer device 50 may be implemented by a plurality of devices, and is an example of the medical image display system.

Communicator 51 obtains captured image 32b from image server 30 through communication. Communicator 51 includes a communication circuit, for example. Communicator 51 is an example of an obtainer.

Receiver 52 includes a graphical user interface (GUI) that receives an instruction (input) from the user. For example, receiver 52 includes input devices, such as a mouse, a keyboard, and a touch panel. Receiver 52 may be configured to receive an instruction from the user by voice or gesture for example.

Controller 53 controls each of the components of viewer device 50. For example, controller 53 performs various kinds of control relating to the medical image displayed by display 54. For example, controller 53 controls a display form of the medical image displayed on display 54, on the basis of captured image 32b obtained from image server 30 and the instruction of the user received from receiver 52. For example, in response to an input received for one of (i) at least one annotation and (ii) at least one structure label (see FIG. 3 described later for example) from the user, controller 53 performs control to change the display form of the other of (i) the at least one annotation and (ii) the at least one structure label. Here, the structure label refers to an ID or name for identifying the anatomical structure.

Under the control of controller 53, display 54 displays captured image 32b obtained from image server 30. Examples of display 54 include, but are not limited to, a liquid crystal display.

More specifically, communicator 51, receiver 52, controller 53, and display 54 included in viewer device 50 are implemented by at least one computer that includes: a nonvolatile memory that holds a program; a volatile memory as a temporary working space; a processor that executes the program; and an input-output circuit that includes a communication interface and a communication port.

Furthermore, communicator 51 and controller 53 included in viewer device 50 may be implemented by a single computer or image display device, or may be implemented separately by a plurality of computers or image display devices that are connected via a communication channel.

Diagnostic support system 10 includes imaging device 20, image server 30, abnormality detection system 40, and viewer device 50 that are connected via the communication channel. Here, some or all of these components may be combined into a single device.

Hereinafter, examples of anatomical structures according to the present disclosure are described with reference to FIG. 2A to FIG. 2D. FIG. 2A to FIG. 2D illustrate examples of anatomical structures (seven different kinds of anatomical structures in total). To be more specific, FIG. 2A to FIG. 2D illustrate, as the examples of the anatomical structures, right atrial shadow and left ventricular shadow, descending aorta shadow, right diaphragmatic dome shadow and left diaphragmatic dome shadow, and outer side of right lung and outer side of left lung. Each of tables in FIG. 2A to FIG. 2D is an example showing, starting from the left, Chest X-ray image, Name of anatomical structure, Area of anatomical structure, and Superimposition of chest X-ray image and anatomical structure. The example of the superimposition of chest X-ray image and anatomical structure is an example of the result of the detection performed by detector 42.

Examples of the anatomical structure on the chest X-ray image include the right atrial shadow, the left ventricular shadow, the descending aorta shadow, and the diaphragmatic dome shadow as illustrated in FIG. 2A to FIG. 2C. The right atrial shadow indicates a boundary extracted between the right atria and its surrounding area on the chest X-ray image. The left ventricular shadow indicates a boundary extracted between the left ventricle and its surrounding area on the chest X-ray image. The descending aorta shadow indicates a boundary extracted between the descending aorta and its surrounding area on the chest X-ray image. The diaphragmatic dome shadow indicates a boundary extracted between the diaphragm and its surrounding area on the chest X-ray image.

Note that the anatomical structures are not limited to these. For example, the anatomical structure may be a lung outer edge that is a boundary extracted between the outer part of the lung and its surrounding area on the chest X-ray image. Alternatively, the anatomical structure may be a different structure or organ, for example. Furthermore, the anatomical structure is not limited to a structure or the like included in the chest X-ray image, and may be a structure or the like included in a medical image, such as a CT image or MRI image captured by a different imaging device.

### [1-2. Construction of Medical Image]

Next, a construction of a medical image displayed by display 54 is described with reference to FIG. 3 and FIG. 4. FIG. 3 illustrates an example of the medical image displayed by display 54 according to the present embodiment. In FIG. 3, different display forms are illustrated by different hatch patterns. The following describes the case where an abnormality is detected in the trachea shadow, which is an example of the anatomical structure. Note that the medical image described below is displayed by display 54 under the control of controller 53.

As illustrated in FIG. 3, the medical image includes processed image 54a (Analysis result in FIG. 3), comparison image 54b (Comparison image in FIG. 3), structure label field 54c (Structure label in FIG. 3), and analysis result field 54d (Analysis result display in FIG. 3). Each of processed image 54a and comparison image 54b is an image included in captured image 32b obtained from image server 30. Processed image 54a, comparison image 54b, structure label field 54c, and analysis result field 54d are displayed at the same time in one medical image. A display area for processed image 54a is an example of a first display area. A display area for structure label field 54c is an example of a second display area. A display area for comparison image 54b is an example of a third display area. A display area for analysis result field 54d is an example of a fourth display area. At least the first display area and the second display area may be areas that do not overlap one another in the medical image. For example, the first to fourth display areas may be areas that do not overlap one another on the medical image. This enhances visibility of the medical image. If viewer device 50 uses a window system, the first to fourth display areas may be displayed in one window or separately displayed in two or more windows.

Processed image 54a indicates a result of detection performed by detector 42. For example, processed image 54a is captured image 32b of the subject. On processed image 54a, at least one segmentation indicating at least one anatomical structure shown in captured image 32b is superimposed on captured image 32b. In processed image 54a illustrated as an example in FIG. 3, information indicating a first reference structure, a second reference structure, a normal structure, and an abnormal structure is superimposed on the chest X-ray image. Here, the segmentation indicates area data obtained as a result of segmentation processing performed on captured image 32b (a segmentation result).

Each of the first reference structure and the second reference structure is an anatomical structure (reference structure) that is not a target of abnormality detection performed by abnormality detection system 40, and indicates a range (structural reference) of detection performed by abnormality detection system 40. Each of the first reference structure and the second reference structure is a structural reference for at least one anatomical structure included in processed image 54a. The first reference structure may indicate a possible approximate upper limit location where a target organ (such as a lung) can be located. The second reference structure may indicate a possible approximate lower limit location where this target organ (such as a lung) can be located. In other words, the first reference structure and the second reference structure indicate a range where the user should perform checking with particular attention in diagnostic imaging. In the present embodiment, the first reference structure and the second reference structure indicate a range where the user should perform checking with particular attention in diagnostic imaging for the chest. In the present embodiment, the first reference structure indicates a first dorsal vertebra shadow and the second reference structure indicates a posterior lung base shadow. However, this is not intended to be limiting.

The normal structure indicates an anatomical structure (normal structure) in which no abnormality is detected by abnormality detection system 40. The abnormal structure indicates an anatomical structure (abnormal structure) in which an abnormality is detected by detector 42. If no abnormality is detected by abnormality detection system 40, no abnormal structure is displayed.

Note that each of the first reference structure, the second reference structure, the normal structure, and the abnormal structure indicates a structure unless otherwise stated.

The following describes display forms of the first reference structure, the second reference structure, the normal structure, and the abnormal structure.

Each of segmentations indicating the first reference structure, the second reference structure, the normal structure, and the abnormal structure is displayed in a different display form. The first reference structure and the second reference structure are used as references and thus displayed unobtrusively by lines thinner than those of the other structures. For example, the first reference structure and the second reference structure are displayed in a color (for example, an achromatic color) visually less prominent than colors of the other structures. More specifically, the first reference structure and the second reference structure are displayed in white (that is, displayed through brightness adjustment). In other words, the display form of the first reference structure and the second reference structure can appear to be less significant than those of the normal structure and the abnormal structure.

The normal structure is displayed in a color that conveys an impression of normality (for example, a chromatic color), or more specifically, displayed in blue (corresponding to the negatively sloping hatch lines in FIG. 3). Furthermore, the normal structure is displayed by a plane or a line thicker than those of the first and second reference structures. For example, if a plurality of anatomical structures are determined to be normal, the plurality of anatomical structures determined to be normal may be displayed in the same display form. More specifically, if the plurality of anatomical structures are determined to be normal, the segmentations corresponding to the plurality of anatomical structures are displayed in blue. Blue is an example of a first display color.

The abnormal structure is displayed in a color that catches attention (for example, a chromatic color), or more specifically, displayed in yellow (corresponding to the positively sloped hatch lines in FIG. 3). Furthermore, the abnormal structure is displayed by a plane or a line thicker than that of the normal structure. For example, if a plurality of anatomical structures are determined to be abnormal, the plurality of anatomical structures determined to be abnormal may be displayed in the same display form. More specifically, if the plurality of anatomical structures are determined to be abnormal, the segmentations corresponding to the plurality of anatomical structures are displayed in yellow. Yellow is an example of a second display color.

In this way, the display color is determined according to the result of the detection performed by abnormality detection system 40, that is, according to the presence or absence of the abnormality, instead of according to the kind of the anatomical structure.

As described above, the two colors, blue and yellow, are used as the display colors and, for example, three or more colors are not used for color-coding. This allows display 54 to display processed image 54a with consideration for a user with color weakness. Furthermore, the small number of display colors reduces the amount of information of processed image 54a, which allows the user to smoothly understand processed image 54a. Moreover, the use of the two colors, blue and yellow, allows the user to intuitively see the normal structure and the abnormal structure.

At least one of the normal structure and the abnormal structure may be displayed with light-transmittance. More specifically, at least one of the normal structure and the abnormal structure may be displayed so that the captured image of the at least one appears light-transmitting. For example, at least the abnormal structure, out of the normal structure and the abnormal structure, may be displayed with light-transmittance. Alternatively, the normal structure and the abnormal structure may be displayed with different transparencies. For example, the light-transmittance of the abnormal structure may be set higher than that of the normal structure.

As a result, an original image can be interpreted simply by viewing processed image 54a.

The different display forms of the first reference structure, the second reference structure, the normal structure, and the abnormal structure are not limited to the different display colors. The first reference structure, the second reference structure, the normal structure, and the abnormal structure only have to be displayed in different display forms. For example, the display forms may have different line forms (such as a solid line, a broken line, and an alternate long and short dash line), or may be different in the state between blinking and non-blinking. Alternatively, the display forms may be different using the combination of these.

Comparison image 54b is used for comparison with processed image 54a for purposes of checking. For example, comparison image 54b is captured image 31b captured before abnormality detection system 40 performs abnormality detection. In the present embodiment, comparison image 54b is captured image 31b (original image) before inputted to the learned model. Comparison image 54b is captured image 31b captured by imaging device 20, for example. Here, comparison image 54b need not be displayed. For example, comparison image 54b need not be displayed if processed image 54a blinks when displayed.

Processed image 54a and comparison image 54b are displayed side by side on one screen. The display of processed image 54a and comparison image 54b on one screen allows the user to make a diagnosis by comparing attention points between processed image 54a and comparison image 54b. Here, an area in which processed image 54a and comparison image 54b are displayed is an example of an image display area.

Note that comparison image 54b is not limited to a captured image corresponding to processed image 54a. Comparison image 54b may be a captured image of the same part of the patient in the past (a year ago, for example). In this case, the user can make a diagnosis by considering the presence or absence of an abnormality in the captured image in the past. Furthermore, comparison image 54b may be an image (a CT image, for example) that captures the user with a device different from the device used to capture processed image 54a. In this case, the user can make a diagnosis by considering the presence or absence of an abnormality in the image captured by the different device.

The number of comparison images 54b displayed at one time is not limited to a particular value, and may be two or more. For example, two images captured at different times in the past (six months ago and one year ago, for example) may be displayed as comparison images 54b at one time. In this case, one of these two images that is captured more recently in the past may be displayed next to processed image 54a. Alternatively, one of these two images that is captured earlier in the past may be displayed next to processed image 54a.

Note that comparison image 54b is not limited to an image of the patient who is captured in processed image 54a. For example, comparison image 54b may be an image of a different patient who shares similar signs or symptoms with the present patient.

FIG. 3 illustrates an example of comparison image 54b obtained before abnormality detection system 40 performs abnormality detection. However, this is not intended to be limiting. Comparison image 54b may be an image obtained as a result of abnormality detection performed by abnormality detection system 40 (for example, an image obtained as a result of abnormality detection performed on an image captured in the past by abnormality detection system 40). If comparison image 54b is an image obtained before abnormality detection system 40 performs abnormality detection, controller 53 may cause abnormality detection system 40 to perform abnormality detection on this image.

Structure label field 54c displays at least one structure label to identify at least one anatomical structure on which abnormality detection system 40 is capable of performing abnormality detection. For example, structure label field 54c displays a structure label indicating an anatomical structure that can be extracted by the learned model used by detector 42. In terms of easy familiarization of the user with the abnormality detection function of abnormality detection system 40, structure label field 54c may display all anatomical structures on which abnormality detection system 40 can is capable of performing abnormality detection.

In the present embodiment, structure label field 54c displays six structure labels, "Trachea shadow", "Descending aorta shadow", "Right atrial shadow" "Left ventricular shadow", "Right diaphragmatic dome shadow", and "Left diaphragmatic dome shadow". Thus, simply by looking at structure label field 54c, the user can see the anatomical structures on which abnormality detection system 40 is capable of performing abnormality detection.

If receiver 52 does not receive, from the user, selection of a segmentation in processed image 54a (selection of an anatomical structure that the user wishes to check), structure label field 54c displays only the structure labels. More specifically, structure label field 54c does not display any information, such as a measurement result, that is different from the structure labels.

A display form of processed image 54a is associated with a display form of structure label field 54c, for example. The display form of an abnormal structure in processed image 54a (for example, the positively sloped hatch lines of the trachea shadow) is associated with the display form of the structure label indicating this abnormal structure in structure label field 54c (the positively sloped hatch lines of trachea shadow 54c1 of the structure label). For example, if an abnormal structure is displayed in yellow in processed image 54a, the structure label indicating this abnormal structure is also displayed in yellow. For example, the display form of an abnormal structure in processed image 54a is identical to the display form of the structure label indicating this abnormal structure.

Thus, the user can easily find the structure label corresponding to the abnormal structure in processed image 54a among a plurality of structure labels. More specifically, the user can find out which one of the anatomical structures is abnormal simply by looking at structure label field 54c.

For example, if a normal structure is displayed in blue in processed image 54a, the structure label indicating this normal structure is also displayed in blue. Thus, the user can easily find the structure label corresponding to the normal structure in processed image 54a among the plurality of structure labels. More specifically, the user can find out which one of the anatomical structures is normal simply by looking at structure label field 54c.

Analysis result field 54d of the medical image displays a result of analyzing a result of the detection performed by detector 42. To be more specific, analysis result field 54d displays a result of the analysis performed on an abnormality of at least one anatomical structure by analyzer 43. For example, analysis result field 54d displays information indicating, based on the detection result, a reason why the anatomical structure is determined to be abnormal.

If receiver 52 has not received selection of a segmentation or a structure label of processed image 54a from the user, analysis result field 54d does not display the analysis result. Note that analysis result field 54d need not be displayed.

Note that the layout, shape, and size used in the medical image illustrated in FIG. 3 may be previously stored in viewer device 50 or may be obtained from, for example, image server 30.

If receiver 52 receives, from the user, an input for one of the segmentation and the structure label of processed image 54a in the medical image illustrated in FIG. 3, controller 53 controls display 54 to change the display form of the other of the segmentation and the structure label in response to the input. The medical image including structure label 54a in the display form that is changed in response to the input for the segmentation from the user is described with reference to FIG. 4. FIG. 4 illustrates an example of the medical image shown when the trachea shadow (see the the positively sloped hatch lines in FIG. 4) is selected among the segmentations. For example, the selection may be made through an easy operation, such as a click operation or a touch operation.

As illustrated in FIG. 4, if receiver 52 receives the selection of the segmentation from the user, display 54 displays (additionally displays) measurement value 54c2 of the anatomical structure corresponding to this segmentation, within structure label field 54c. For example, among measurement values of the anatomical structures, display 54 displays only measurement value 54c2 of the anatomical structure corresponding to the segmentation selected by the user, within structure label field 54c. At this time, display 54 may change at least one of the position or the size of at least one of a plurality of structure labels. Note that displaying (additionally displaying) measurement value 54c2 in structure label field 54c is an example of changing the display form of at least one of the structure labels.

Display 54 displays measurement value 54c2 in a position near the structure label corresponding to the segmentation selected by the user (for example, in a position adjacent to this structure label). However, display 54 may display (additionally display) measurement value 54c2 within the structure label corresponding to the segmentation selected by the user. Displaying, by display 54, measurement value 54c2 in the position near or within the corresponding structure label is an example of additionally displaying measurement value 54c2 to the structure label corresponding to the segmentation.

In the example of FIG. 4, receiver 52 receives the selection of the segmentation indicating an abnormal structure (for example, the trachea shadow) from the user, and thus display 54 displays only measurement value 54c2 related to trachea shadow 54c1 of structure label field 54c. Furthermore, display 54 displays measurement value 54c2 in the display form identical to the display form of this structure label. In the present embodiment, display 54 displays measurement value 54c2 in yellow (a yellow background). Thus, display 54 can display the measurement value only when needed by the user.

If receiver 52 further receives selection of a different segmentation from the user, display 54 may hide the measurement value corresponding to the previously-selected segmentation or may keep this measurement value displayed.

If receiver 52 receives selection of trachea shadow 54c1 that is a structure label, display 54 may switch between displaying and hiding the segmentation indicated by this structure label in processed image 54a. Alternatively, display 54 may highlight this segmentation when displayed. Furthermore, if receiver 52 receives this selection from the user, display 54 may display measurement value 54c2 corresponding to the selected structure label, near this structure label.

As described above, the display forms of processed image 54a and structure label field 54c are linked with each other. Thus, if one of processed image 54a and structure label field 54c is selected by the user, the image in the other of processed image 54a and structure label field 54c changes in response to this selection.

Furthermore, if receiver 52 further receives selection of a segmentation from the user, display 54 may display a result of analysis performed on the anatomical structure corresponding to this segmentation by analyzer 43, in analysis result field 54d. To be more specific, along with the selection of the segmentation by the user, or more specifically, along with displaying of measurement value 54c2 corresponding to the selected segmentation, display 54 may display the result of the analysis performed on the anatomical structure corresponding to this selected segmentation by analyzer 43.

Analysis result field 54d displays only the analysis result of the anatomical structure corresponding to the segmentation selected by the user. Analysis result field 54d displays the analysis result in text. For example, "Unclear due to inadequate detection of trachea shadow" is displayed as the analysis result. Here, "Unclear due to inadequate detection of trachea shadow" means that an area of the segmentation corresponding to the trachea shadow is smaller than a predetermined area (or more specifically, the area is insufficient).

Note that if receiver 52 receives selection of a structure label in structure label field 54c from the user, display 54 may similarly display, in analysis result field 54d, a result of analysis performed on the anatomical structure corresponding to the selected structure label by analyzer 43.

In this way, the display forms of processed image 54a and structure label field 54c are linked with the display form of analysis result field 54d. If the user selects one of processed image 54a and structure label field 54c, the image in the other of processed image 54a and structure label field 54c changes in response to this selection. Furthermore, the analysis result of the anatomical structure corresponding to this selection may be displayed in analysis result field 54d. This control is performed by controller 53.

Thus, only the analysis result that the user wished to check for making a diagnosis can be displayed. On this account, the user can effectively make the diagnosis, as compared to when a plurality of analysis results are displayed.

### [1-3. Operation of Diagnostic Support System]

Next, to describe an operation of diagnostic support system 10 according to the present embodiment described above, an operation of viewer device 50, which is a characteristic component in diagnostic support system 10, is mainly described.

FIG. 5 is a flowchart of a main operation (or more specifically, a medical image display method) performed by viewer device 50 according to the present embodiment. Note that FIG. 5 illustrates an operation performed to change the display form of the structure label when selection of a segmentation is received.

As illustrated in FIG. 5, communicator 51 first obtains captured image 32b (the analysis result and the comparison image illustrated in FIG. 3) from image server 30 (S10). For example, communicator 51 obtains processed image 54a and comparison image 54b as captured image 32b. Processed image 54a and comparison image 54b may be obtained at the same time or at different times. Captured image 32b may be obtained in response to an operation performed by the user using receiver 52 of viewer device 50. Alternatively, captured image 32b may be obtained whenever image server 30 obtains captured image 32a from abnormality detection system 40 after the abnormality detection. Furthermore, if viewer device 50 stores comparison image 54b, controller 53 may obtain comparison image 54b from a storage device of viewer device 50.

Controller 53 determines whether an abnormal structure is present in processed image 54a of captured image 32 obtained (S20). For example, controller 53 may determine whether an abnormal structure is present, on the basis of at least one of the detection result or the analysis result provided by abnormality detection system 40 and included in the data outputted from abnormality detection system 40. For example, controller 53 may determine whether the abnormal structure is present, on the basis of a result of determination whether an abnormality is included in the analysis result.

If an abnormal structure is present in processed image 54a (Yes in S20), controller 53 decides to use the display form for the abnormal structure that is different from the display form of a normal structure (S30). For example, controller 53 determines that the display forms of the abnormal structure and the normal structure are to be different from each other, or more specifically, determines the display form according to the result of the determination made by abnormality detection system 40 whether the abnormality is present. In other words, controller 53 determines the display form for each of the abnormal structure and the normal structure, regardless of the kind of the anatomical structure. In the present embodiment, controller 53 determines that the abnormal structure is to be displayed in yellow that catches attention and that the normal structure is to be displayed in blue that conveys the impression of normality.

For example, controller 53 may determine the display colors using a table that associates the presence or absence of the abnormal structure with a display color.

Furthermore, controller 53 may further determine the display form of the first reference structure and the second reference structure in Step S30. For the first reference structure and the second reference structure, controller 53 may determine the display form that is different from each of the display forms of the abnormal structure and the normal structure and that makes these reference structures visually less prominent than the other structures in processed image 54a. For example, controller 53 may display the first reference structure and the second reference structure in white.

Furthermore, controller 53 may further determine the display form of the structure labels in structure label field 54c according to processed image 54a. For example, regardless of the anatomical structure, controller 53 may make the determination so that the display form of the structure label indicating the abnormal structure is identical to the display form of the segmentation indicating the abnormal structure in processed image 54a, and that the display form of the structure label indicating the normal structure is identical to the display form of the segmentation indicating the normal structure in processed image 54a.

If an abnormal structure is not present in processed image 54a (No in S20), controller 53 proceeds to Step S40.

Next, controller 53 displays the analysis result and the comparison image (or more specifically, processed image 54a and comparison image 54b) on one screen (S40). For example, controller 53 causes display 54 to display the medical image illustrated in FIG. 3. To be more specific, controller 53 displays, on display 54, the medical image including processed image 54a, comparison image 54b, structure label field 54c, and analysis result field 54d, for example.

Next, controller 53 determines whether selection of a segmentation (segmentation display) is received from the user via receiver 52 (S50). If the selection of the segmentation is received from the user via receiver 52 (Yes in S50), controller 53 displays the measurement result and the analysis result relating to the received selection of segmentation (S60). If it is determined as "Yes" in Step S50, controller 53 displays the measurement result (measurement value 54c2, for example) of the anatomical structure corresponding to the received selection of the segmentation, at a position where the measurement result does not overlap with processed image 54a and comparison image 54b. In the present embodiment, controller 53 displays this measurement result in structure label field 54c. Furthermore, if it is determined as "Yes" in Step S50, controller 53 according to the present embodiment further displays the analysis result of the anatomical structure corresponding to the received selection of the segmentation, in analysis result field 54d. More specifically, controller 53 displays the medical image illustrated in FIG. 4 in Step S60. In other words, the display form of the medical image displayed on display 54 changes from the display form illustrated in FIG. 3 to the display form illustrated in FIG. 4.

For example, controller 53 displays the measurement value, which is an example of the measurement result, near the structure label corresponding to the selected segmentation. At the same time, if this structure label indicates an abnormality, controller 53 displays the analysis result in analysis result field 54d. For example, the measurement value includes the position ("Position" in FIG. 4), the area ("Area" in FIG. 4), and the presence or absence of fragments ("Fragment" in FIG. 4) of the anatomical structure extracted by detector 42. Note that the fragments indicate that a line of the shadow is detected as a plurality of fragments, for example. If the fragments are detected, it is determined that an abnormality is present.

In this way, when needed by the user, display 54 displays information necessary for the user to make a diagnosis. Thus, display 54 can reduce display of information that is unnecessary for the user to make a diagnosis. This can prevent a decrease in the diagnostic efficiency.

Furthermore, diagnostic support system 10 according to the present embodiment may perform display switching between a full-structure display mode and a selected-structure display mode, as described below with reference to FIG. 6. The display described below may be shown as in FIG. 6. FIG. 6 is a state transition diagram illustrating transitions of the display state of viewer device 5 according to Embodiment 1. Note that the operation illustrated in FIG. 6 is not essential.

Note that all of the segmentations in processed image 54a illustrated in FIG. 3 blink in a full-structure blinking display in initial state I1. The interval of blinking may have a length that allows for a diagnosis of an abnormal structure. Examples of the interval include, but are not limited to, a few seconds. Initial state I1 is an example of a first display mode.

In initial state I1, full functions (structure labels, for example) of detector 42 are presented. This prevents the user from overlooking, and is expected to have an effect of enhancing understanding of the user. Furthermore, blinking of the segmentations allows the user to interpret the original image without any operation. Note that the blinking display is not essential in initial state I1.

For example, a predetermined operation (such as a mouse click) performed in the state illustrated in FIG. 3 may enable the blinking display. Note that the display illustrated in FIG. 6 is provided for processed image 54a illustrated in FIG. 3 and that the display of comparison image 54b does not change. To be more specific, the operation described below does not change the display form of comparison image 54b.

As illustrated in FIG. 6, if receiving an operation performed to switch to the full-structure display mode (a first operation) from the user via receiver 52 in initial state I1, display 54 switches initial state I1 to the full-structure display mode to provide full-structure display I2a (S70). The full-structure display mode displays an image where the blinking display of the segmentations is stopped in initial state I1. More specifically, all of the segmentations are displayed all the time (displayed continuously, for example) in the full-structure display mode. The full-structure display mode is an example of a third display mode.

If further receiving a second operation from the user via receiver 52, display 54 switches full-structure display I2a to original image I2b (S71). In the original image, the segmentations (annotations) are hidden from full-structure display I2a. The original image is obtained by hiding the segmentations from processed image 54a illustrated in FIG. 3. If comparison image 54b is captured image 31b before inputted to the learned model, comparison image 54b is identical to original image I2b.

If further receiving the second operation via receiver 52, display 54 switches original image I2b to full-structure display I2a (S72). Whenever the second operation is performed, display 54 switches between full-structure display I2a and original image I2b. If receiving an operation to stop the full-structure display mode (a third operation) via receiver 52, display 54 switches the full-structure display mode to initial state I1 (S73).

Note that each of the first to third operations may be a predetermined operation performed with the keyboard or the mouse, for example. For example, each of the first to third operations may be performed with a predetermined key on the keyboard. The first operation and the second operation may be the same operation (the same key operation). The second operation for the switching in Step S71 and the second operation for the switching in Step S72 may be the same operation (the same key operation) or different operations.

If receiving selection of a segmentation or a structure label (a fourth operation) from the user via receiver 52 in initial state I1, display 54 switches initial state I1 to the selected-structure display mode to provide selected-structure display I3a (S80). The selected-structure display mode highlights the segmentation selected by the user. In the present embodiment, the selected-structure display mode causes the segmentation selected by the user to blink and hides the other segmentations. More specifically, only the segmentation selected by the user blinks in the selected-structure display mode. The selected-structure display mode is an example of a second display mode.

If further receiving a fifth operation via receiver 52, display 54 switches selected-structure display I3a to original image I3b (S81). In the original image, the segmentations (annotations) are hidden from selected-structure display I3a. The original image is obtained by hiding the segmentations from processed image 54a illustrated in FIG. 3. The original image I3b may be identical to original image I2b, for example.

If further receiving the fifth operation via receiver 52, display 54 switches original image I3b to selected-structure display I3a (S82). Whenever the fifth operation is performed, display 54 switches between selected-structure display I3a and original image I3b. If receiving an operation to stop the selected-structure display mode (a sixth operation) via receiver 52, display 54 switches the selected-structure display mode to initial state I1 (S83).

Note that each of the fourth to sixth operations may be a predetermined operation performed with the keyboard or the mouse, for example. For example, each of the fourth to sixth operations may be performed with a predetermined key on the keyboard. The fifth operation for the switching in Step S81 and the fifth operation for the switching in Step S82 may be the same operation (the same key operation) or different operations. Furthermore, the fourth operation and the fifth operation may be the same operation (the same key operation), and the fifth operation and the second operation may be the same operation. Moreover, the sixth operation and the third operation may be the same operation (the same key operation).

If receiving a seventh operation via receiver 52 in the full-structure display mode, display 54 may switch the full-structure display mode to the selected-structure display mode (S90). Furthermore, if receiving an eighth operation via receiver 52 in the selected-structure display mode, display 54 may switch the selected-structure display mode to the full-structure display mode (S91). Note that the seventh operation and the eighth operation may be the same operation.

As described above, viewer device 50 may change the display form of processed image 54a in response to an operation performed by the user using receiver 52. Thus, a predetermined operation performed by the user using receiver 52 enables viewer device 50 to provide a necessary amount of information when needed by the user. This prevents the user from being interrupted during interpretation.

Some of the first to eighth operations are the same. This allows viewer device 50 to easily change the display state without performing different types of operations.

Note that the display switching in response to the predetermined operation performed using receiver 52 is not limited to the above. For example, in response to a predetermined operation, viewer device 50 may switch between displaying and not displaying at least one of comparison image 54b, structure label field 54c, or analysis result field 54d included in the medical image. To be more specific, the operation performed using receiver 52 may hide at least one of comparison image 54b, structure label field 54c, or analysis result field 54d.

Furthermore, viewer device 50 may change a light-transmittance level of a segmentation of a planar structure (such as the trachea shadow) or a linear structure (such as the five anatomical structure shadows other than the trachea shadow), in response to a predetermined operation for example. Controller 53 of viewer device 50 may change the light-transmittance level of a segmentation in response to an input received for one of the segmentation indicating an abnormal structure and the structure label corresponding to this segmentation.

The light-transmittance level of the planar structure or the linear structure is 50% in the initial state, for example. Then, the light-transmittance level may gradually change to 80% and to 100% (hidden) in response to the predetermined operation. This allows the user to check a different organ that overlaps with the segmentation.

Furthermore, viewer device 50 may change the color of at least one of the first display color or the second display color, in response to a predetermined operation for example. For example, controller 53 of viewer device 50 may change the color of at least one of the first display color or the second display color in response to an input received for one of the segmentation and the at least one structure label. For example, in response to an input received for one of a segmentation indicating an abnormal structure and the structure label corresponding to this segmentation, controller 53 may change the display color of the segmentation indicating the abnormal structure. Moreover, in response to an input received for one of a segmentation indicating a normal structure and the structure label corresponding to this segmentation, controller 53 may change the display color of the segmentation indicating the normal structure. Note that, with the change of the display color of the segmentation, the display color of the structure label corresponding to this segmentation may also be changed.

Furthermore, viewer device 50 may cause the segmentation (an example of the annotation) of a planar structure to be solidly shaded or not, in response to a predetermined operation for example. Without solid shade on the segmentation of the planar structure, viewer device 50 can show the contour of the planar structure. This allows the user to check a different organ that overlaps with the segmentation.

Furthermore, viewer device 50 may change the line width of the segmentation (an example of the annotation) of a linear structure, in response to a predetermined operation for example. Such variations of the line width of the linear structure allow the user to choose the line width suitable for the interpretation.

Note that examples of the predetermined operation include, but are not limited to, an easy operation like mouse dragging or a key operation.

### [1-4. Advantageous effects etc.]

As described thus far, viewer device 50 (an example of the medical image display system) according to the present embodiment is a medical image display system that displays a medical image. The medical image includes: a first display area for displaying processed image 54a in which at least one annotation indicating a result of detection of at least one anatomical structure shown in captured image 32b of a subject is superimposed on captured image 32b; and a second display area (structure label field 54c, for example) for displaying at least one structure label for identifying the at least one anatomical structure. Viewer device 50 includes: display 54 that displays the medical image; and controller 53 that changes, in response to an input received for one of (i) the at least one annotation and (ii) the at least one structure label from the user, a display form of the other of (i) the at least one annotation and (ii) the at least one structure label.

With this, viewer device 50 allows the user to recognize which one of the anatomical structures has its detection result displayed in processed image 54a simply by taking one glance at the structure label displayed in the second display area. Thus, viewer device 50 can prevent the user from overlooking the anatomical structure and also enhance understanding of the user about the anatomical structure. Hence, viewer device 50 can prevent a decrease in the diagnostic efficiency when the user makes a diagnosis using processed image 54a. In other words, viewer device 50 can enhance the diagnostic efficiency when the user makes a diagnosis using processed image 54a.

Furthermore, in response to an input of selection of a first annotation among the at least one annotation, controller 53 causes a measurement result of the anatomical structure corresponding to the first annotation to be additionally displayed in the structure label corresponding to the first annotation.

With this, controller 53 can display the measurement result only when needed by the user. For example, controller 53 can reduce the amount of information to be displayed, as compared to when the measurement result of the anatomical structure is displayed all the time. This enhances the understanding of the user about the measurement result. Hence, viewer device 50 can prevent a decrease in the diagnostic efficiency when a diagnosis is made using the measurement result.

Furthermore, in response to an input of selection of a first structure label among the at least one structure label, controller 53 causes display 54 to switch between displaying and not displaying the annotation corresponding to the first structure label.

With this, controller 53 can display the annotation only when needed by the user. For example, controller 53 can reduce the amount of information to be displayed, as compared to when the annotation is displayed all the time. This enhances the understanding of the user about processed image 54a. Hence, viewer device 50 can prevent a decrease in the diagnostic efficiency when a diagnosis is made using processed image 54a.

Furthermore, the medical image further includes a third display area for displaying comparison image 54b to be compared with processed image 54a.

With this, viewer device 50 can display both processed image 54a and comparison image 54b on one screen. This allows the user to easily compare processed image 54a with comparison image 54b. Hence, viewer device 50 can prevent a decrease in the diagnostic efficiency when a diagnosis is made by comparison between processed image 54a and comparison image 54b.

Furthermore, the medical image further includes a fourth display area (for example, analysis result field 54e) for displaying a result of analysis performed on an abnormality in the at least one anatomical structure.

With this, viewer device 50 allows the user to make a diagnosis using the analysis result. The diagnosis made using the analysis result is expected to enhance the diagnostic efficiency for the user.

Furthermore, in response to an input received for one of (i) the at least one annotation and (ii) the at least one structure label, controller 53 causes a result of analysis performed on the anatomical structure identified based on this input to be displayed in the fourth display area.

With this, controller 53 can display the analysis result only when needed by the user. For example, controller 53 can reduce the amount of information to be displayed, as compared to when the analysis result of the anatomical structure is displayed all the time. This enhances the understanding of the user about the analysis result. Hence, viewer device 50 can prevent a decrease in the diagnostic efficiency when a diagnosis is made using the analysis result.

Furthermore, the at least one annotation includes (i) a segmentation indicating an abnormal structure among the at least one anatomical structure and (ii) a segmentation indicating a normal structure among the at least one anatomical structure. The segmentation indicating the abnormal structure is light-transmissive.

With this, viewer device 50 allows the user to interpret the original image using processed image 54a. Hence, viewer device 50 enables easy interpretation of the original image and thus prevents a decrease in the diagnostic efficiency.

Furthermore, in response to an input received for (i) one of the segmentation indicating the abnormal structure and (ii) the structure label corresponding to this segmentation, controller 53 causes the light-transmittance level of this segmentation to be changed.

With this, controller 53 can change the light-transmittance level of the segmentation to a light-transmittance level desired by the user. To be more specific, controller 53 has the function of presenting processed image 54a that can be easily interpreted by the user. Hence, viewer device 50 further prevents a decrease in the diagnostic efficiency.

Furthermore, the at least one annotation includes (i) a segmentation indicating an abnormal structure among the at least one anatomical structure and (ii) a segmentation indicating a normal structure among the at least one anatomical structure. The segmentation indicating the normal structure and the structure label corresponding to this segmentation are displayed in a first display color. The segmentation indicating the abnormal structure and the structure label corresponding to this segmentation are displayed in a second display color different from the first display color.

With this, viewer device 50 can display the result of the detection performed on the anatomical structure, which is a target of the detection of the presence or absence of an abnormality, using the two display colors. Thus, the medical image can be displayed with consideration for a user with color weakness. Hence, even for the user with color weakness, viewer device 50 can prevent a decrease in the diagnostic efficiency.

Furthermore, in response to an input regarding one of (i) the segmentation indicating the abnormal structure and the segmentation indicating the normal structure and (ii) the at least one structure label, controller 53 causes at least one of the first display color or the second display color to be changed.

With this, controller 53 can change the display color of the segmentation to a display color desired by the user. To be more specific, controller 53 has the function of presenting processed image 54a that is easily interpreted by the user. Hence, viewer device 50 can further prevent a decrease in the diagnostic efficiency.

Furthermore, the at least one annotation further includes a segmentation indicating a reference structure which is used as a structural reference of a corresponding one of the at least one anatomical structure in processed image 54a. The segmentation indicating the reference structure is displayed in an achromatic color.

With this, viewer device 50 can display the reference structure without increasing the number of display colors. Hence, even for the user with color weakness, viewer device 50 can further prevent a decrease in the diagnostic efficiency.

Furthermore, the display form includes: a first display mode in which each of the at least one annotation blinks; and a second display mode in which a second annotation corresponding to an input among the at least one annotation blinks and an annotation other than the second annotation is not displayed. In response to an input of selection of the second annotation among the at least one annotation in the first display mode, controller 53 causes the first display mode to be switched to the second display mode.

With this, controller 53 can display processed image 54a in the first display mode or the second display mode that is desired by the user. To be more specific, controller 53 has the function of presenting processed image 54a that can be easily interpreted by the user. Hence, viewer device 50 can further prevent a decrease in the diagnostic efficiency. Moreover, controller 53 causes the annotation to blink when displayed. Thus, the original image interpretable can be displayed without the user having to perform an operation.

As described thus far, the medical image display method according to the present embodiment is a medical image display method that displays a medical image. The medical image includes: a first display area for displaying processed image 54a in which at least one annotation indicating a result of detection of at least one anatomical structure shown in captured image 32b of a subject is superimposed on captured image 32b; and a second display area (structure label field 54c, for example) for displaying at least one structure label for identifying the at least one anatomical structure. Moreover, in response to an input received for one of (i) the at least one annotation and (ii) the at least one structure label from the user, the medical image display method changes the display form of the other of (i) the at least one annotation and (ii) the at least one structure label (S60). Furthermore, as described thus far, the program according to the present embodiment may be a program for causing a computer to execute the aforementioned medical image display method.

With this, the method achieves the same advantageous effects as viewer device 50.

### [Embodiment 2]

### [2-1. Configuration of Diagnostic Support System]

FIG. 7 is a block diagram illustrating a configuration of viewer device 50a according to the present embodiment. As a main difference from viewer device 50 according to Embodiment 1, viewer device 50a according to the present embodiment includes image processor 55. The following mainly describes differences between viewer device 50 according to Embodiment 1 and viewer device 50a according to the present embodiment. Components that are identical or similar to the components of viewer device 50 according to Embodiment 1 are assigned the same reference signs as in Embodiment 1. Thus, description on these components is omitted or simplified. Note that the other components included in diagnostic support system 10 may be identical or similar to those in Embodiment 1, and that description on these components is omitted.

In response to an instruction received from the user via receiver 52, viewer device 50a displays captured image 32b stored in image server 30 and various kinds of information associated with captured image 32b, in various display forms. Viewer device 50a includes communicator 51, receiver 52, controller 53, display 54, and image processor 55.

Image processor 55 performs image processing on captured image 32b, on the basis of an amount of anatomical structure (for example, the number of pixels) extracted by detector 42. For example, if the amount of anatomical structure extracted by detector 42 is less than a threshold value, image processor 55 superimposes a probabilistic atlas corresponding to the anatomical structure, the extracted amount of which is less than the threshold value, on captured image 32b. The probabilistic atlas is an anatomical model and indicates a spatial existence probability (probability map) of the anatomical structure on a medical image. The probabilistic atlas can also be an existence probability map indicating an existence probability of the anatomical structure. The probabilistic atlas indicates the existence probability and thus is multivalued.

For example, the probabilistic atlas is generated by modeling a normal anatomical structure. The probabilistic atlas is generated on the basis of multiple previously-obtained normal case images (images of normal anatomical structures), for example. In a specific procedure, a reference image designated by hand (manually) to indicate an area corresponding to a target anatomical structure (an anatomical structure area) in the image is first generated for each of the multiple normal case images. The reference image is also referred to as correct data or training data. Next, the designated anatomical structure area is normalized using the location or size of an anatomical structure like a lung field area that is not the target anatomical structure but can be a reference anatomical structure. Each of multiple reference images based on the multiple normal case images is normalized. Next, the normalized anatomical structure areas are averaged to create an existence probability map that indicates the existence probability of the anatomical structure. This existence probability map corresponds to the probabilistic atlas. Note that the method of generating the probabilistic atlas is not limited to this and that any known method can be used.

In the present embodiment, the probabilistic atlas is generated by modeling each of six normal anatomical structures, "trachea shadow", "descending aorta shadow", "right atrial shadow" "left ventricular shadow", "right diaphragmatic dome shadow", and "left diaphragmatic dome shadow". The probabilistic atlas is previously generated and stored in a storage device of viewer device 50a. However, the probabilistic atlas may be obtained from image server 30, for example.

Image processor 55 performs the aforementioned processing under the control of controller 53, for example. However, this is not intended to be limiting.

In addition to or instead of the control described in Embodiment 1, controller 53 performs control to cause the probabilistic atlas to be superimposed on a processed image. For example, assume that the size of an annotation, among at least one annotation, that indicates an anatomical structure (abnormal structure) detected as having an abnormality is less than the threshold value. In this case, controller 53 causes the probabilistic atlas that indicates a normal structure of this currently abnormal structure (a normal structure assumed when this abnormal structure is normal) to be superimposed on the captured image.

To be more specific, image processor 55 included in viewer device 50a is implemented by at least one computer that includes: a nonvolatile memory that holds a program; a volatile memory as a temporary working space; a processor that executes the program; and an input-output circuit that includes a communication interface and a communication port.

Furthermore, communicator 51, controller 53, and image processor 55 included in viewer device 50a may be implemented by a single computer or image display device, or may be implemented separately by a plurality of computers or image display devices that are connected via a communication channel.

Note that controller 53 may determine whether the amount of anatomical structure extracted by detector 42 is less than the threshold value. Alternatively, controller 53 may obtain a result of the determination from an external device.

### [2-2. Construction of Medical Image]

Next, a construction of a medical image displayed by display 54 is described with reference to FIG. 8A to FIG. 8C. FIG. 8A illustrates a first example of the medical image displayed by display 54 according to the present embodiment. Note that each of FIG. 8A and FIG. 8B illustrates that only the extracted amount of trachea shadow, among the anatomical structures, is less than the threshold value.

FIG. 8A illustrates processed image 154a obtained when the trachea shadow is not detected by detector 42. To be more specific, FIG. 8A illustrates processed image 154a obtained when the extracted amount of trachea shadow (the amount of structure extraction) is less than the threshold value (0 pixels). Processed image 154a is an example of the medical image.

In this case, image processor 55 superimposes probabilistic atlas 154a1 on processed image 154a. Moreover, image processor 55 displays probabilistic atlas 154a1 in a display form different from a display form of a segmentation (for example, a binary display form).

For example, if the segmentation is displayed with no change in at least one of brightness or hue (displayed in the binary display form, for instance), image processor 55 may display probabilistic atlas 154a1 by gradations. For gradational display, at least one of brightness or hue gradually changes. For example, image processor 55 may display probabilistic atlas 154a1 by gradations from a first color (yellow, for instance) to a second color (red, for instance), or from a first brightness (a dark display, for instance) to a second brightness (a display brighter than the first brightness).

Alternatively, image processor 55 may display an area having a probability less than a predetermined value in probabilistic atlas 154a1 by gradations, for example. More specifically, image processor 55 may display a part of probabilistic atlas 154a1 by gradations. FIG. 8A illustrates an example of displaying only the outer edge of probabilistic atlas 154a1 more brightly than the other parts. Moreover, image processor 55 may reflect an existence probability level of the anatomical structure in the gradations of the probabilistic atlas. Image processor 55 may display an area having a higher existence probability more brightly (with a higher brightness) and an area having a lower existence probability more darkly (with a lower brightness). For example, image processor 55 may display the area having a higher existence probability in a warm color and the area having a lower existence probability in a cool color. To be more specific, image processor 55 may show a display like a so-called heat map.

For example, if the segmentation has no light-transmittance, image processor 55 may display probabilistic atlas 154a1 with light-transmittance. If the segmentation has light-transmittance, image processor 55 may display probabilistic atlas 154a1 with no light-transmittance.

With this, when probabilistic atlas 154a1 is superimposed on processed image 154a, image processor 55 can prevent the user from misinterpreting probabilistic atlas 154a1 as the segmentation (the structure extracted by detector 42). Moreover, image processor 55 displays the anatomical structure, which is not displayed when the result of the detection from detector 42 is displayed, as probabilistic atlas 154a1. This can prevent the user from overlooking this anatomical structure.

Note that image processor 55 may adjust the size and position of the probabilistic atlas on the basis of the first reference structure and the second reference structure for example. For example, on the basis of at least one of the size or the position of the first reference structure or the second reference structure, image processor 55 may normalize at least one of the size or the position of the probabilistic atlas. Note that the anatomical structure used as a reference for the normalization of the probabilistic atlas is not limited to the first reference structure and the second reference structure. The anatomical structure used as a reference may be any other anatomical structure that can be a reference.

Next, the following describes processed image 154b obtained when detector 42 detects the trachea shadow and the amount of extraction (the amount of structure extraction) is less than the threshold value, with reference to FIG. 8B. FIG. 8B illustrates a second example of the medical image displayed by display 54 according to the present embodiment. In FIG. 8B, a part of the trachea shadow is detected by detector 42 and thus segmentation 154b2 indicating this part of the trachea shadow is included in processed image 154b.

As illustrated in FIG. 8B, image processor 55 superimposes probabilistic atlas 154b1 on processed image 154b so that segmentation 154b2 is visible. For example, if segmentation 154b2 indicating the abnormal structure and probabilistic atlas 154a1 are superimposed on processed image 154b, image processor 55 displays segmentation 154b2 and probabilistic atlas 154a1 so that segmentation 154b2 is visible. In other words, image processor 55 displays an area other than the area extracted by detector 42 (the area of segmentation 154b2) with probabilistic atlas 154a1, for example. The display forms of probabilistic atlas 154a1 and segmentation 154b2 are different. Thus, even if the image includes both probabilistic atlas 154a1 and segmentation 154b2, viewer device 50a can prevent the user from confusing between probabilistic atlas 154a1 and segmentation 154b2.

Next, the following describes processed image 154c obtained when the amount of extraction (the amount of structure extraction) of the trachea shadow by detector 42 is greater than or equal to the threshold value, with reference to FIG. 8C. FIG. 8C illustrates a third example of the medical image displayed by display 54 according to the present embodiment. In FIG. 8C, the amount of the trachea shadow extracted by detector 42 is greater than or equal to the threshold value and thus the probabilistic atlas corresponding to the trachea shadow is not displayed.

As illustrated in FIG. 8C, segmentation 154c1 with pixels corresponding to the threshold value or greater is superimposed on processed image 154c. In this case, the probabilistic atlas is not displayed on processed image 154c and only a plurality of segmentations including segmentation 154c1 are superimposed. For example, processed image 154c illustrated in FIG. 8C may be identical to processed image 54a illustrated in FIG. 3.

With this, when the amount of extraction (the amount of structure extraction) of the trachea shadow by detector 42 is greater than or equal to the threshold value and thus the user is unlikely to overlook the abnormal structure, the information displayed on display 54 can be reduced. This can prevent a decrease in the efficiency of diagnosis made by the user.

Note that the predetermined value may be set for each of the anatomical structures for example, or may be set depending on the number of pixels of display 54 for example.

Note that if operations corresponding to Steps S70 to S73 of FIG. 6 are performed when the processed image including the probabilistic atlas is displayed, controller 53 may change only the display form of the segmentation. Alternatively, controller 53 may change the display forms of the segmentation and the probabilistic atlas, or may change only the display form of the probabilistic atlas. For example, for the transition in Step S71, the image that hides the probabilistic atlas may be displayed as the original image. As with the display form of the segmentation, the display form of the probabilistic atlas may change depending on the selection of the probabilistic atlas. For example, the display form of the probabilistic atlas may switch between blinking or non-blinking. Alternatively, the light-transmittance level of the probabilistic atlas may be variable, or the manner of gradation of the probabilistic atlas may be switchable. Note that when the probabilistic atlas is hidden, this means that the probabilistic atlas is not displayed unless receiver 52 receives a predetermined operation.

### [2-3. Operation of Diagnostic Support System]

Next, to describe an operation of diagnostic support system 10 according to the present embodiment as described above, an operation of viewer device 50a, which is a characteristic component in diagnostic support system 10, is mainly described. FIG. 9 is a flowchart of a main operation (or more specifically, a medical image display method) performed by viewer device 50a according to the present embodiment.

FIG. 9 illustrates, as an example, that viewer device 50a performs Steps S110 and S120 in addition to the operation performed by viewer device 50 according to the embodiment. However, this is not intended to be limiting. Viewer device 50a only has to perform at least Steps S110 and S120. In FIG. 9, steps identical or similar to those in FIG. 5 are assigned the same reference marks used in FIG. 5. Thus, description on these steps is omitted here.

As illustrated in FIG. 9, if an abnormal structure is present in captured image 32b obtained (Yes in S20), controller 53 further determines whether a structure (abnormal structure) with the amount of extraction less than the threshold value is present (S110). For example, controller 53 may determine, for each of the anatomical structures indicated by the structure labels included in structure label field 54c, whether the anatomical structure includes an abnormal structure with the amount of extraction less than the threshold value. Examples of the amount of extraction include, but are not limited to, the number of pixels. For example, controller 53 makes the determination in Step S110 by reading the threshold value for the number of pixels from a storage device (not shown) of viewer device 50a and then comparing the read threshold value with the number of pixels of the segmentation in captured image 32b. Note that the threshold value for the number of pixels is set for each of the anatomical structures. As another example, the amount of extraction may be a ratio obtained by dividing the number of pixels by the standard number of pixels of the anatomical structure.

If a structure (abnormal structure) with the amount of extraction less than the threshold value is present (Yes in S110), controller 53 causes image processor 55 to superimpose the probabilistic atlas on captured image 32b. Image processor 55 reads the probabilistic atlas corresponding to the abnormal structure with the amount of extraction less than the threshold value from a storage device (not shown) of viewer device 50a. Then, image processor 55 superimposes the read probabilistic atlas on captured image 32b. More specifically, image processor 55 superimposes the probabilistic atlas corresponding to the abnormal structure with the amount of extraction less than the threshold value on captured image 32b (S120). Image processor 55 superimposes the probabilistic atlas at a position of this anatomical structure in captured image 32b.

As described above, controller 53 determines whether the size of the segmentation indicating the abnormal structure is less than the threshold value. Then, if determining that the size of the segmentation is less than the threshold value, controller 53 causes the probabilistic atlas corresponding to this abnormal structure to be superimposed on captured image 32b.

If a structure (abnormal structure) with the amount of extraction less than the threshold value is not present (No in S110), controller 53 proceeds to Step S30.

If selection of the probabilistic atlas instead of the segmentation display (segmentation) is received in Step S50, controller 53 may display information relating to the received selection of the probabilistic atlas and an analysis result on display 54 in Step S60.

The information relating to the probabilistic atlas may be the size or number of pixels of the probabilistic atlas, for example. Alternatively, the information may indicate that the probabilistic atlas is being displayed. Furthermore, the analysis result may be the number of pixels of the segmentation indicating the abnormal anatomical structure.

Viewer device 50a may cause display 54 to display processed images 154a to 154c as the medical image. Alternatively, viewer device 50a may cause display 54 to display the medical image including processed images 154a to 154c, instead of processed image 54a illustrated in FIG. 3. For example, viewer device 50a may cause display 54 to display the medical image including: one of processed images 154a to 154c; comparison image 54b; structure label field 54c; and analysis result field 54d.

### [2-4. Advantageous effects etc.]

As described above, viewer device 50a (an example of a medical image display system) according to the present embodiments displays a medical image. The medical image includes processed image 154a and the like in which at least one annotation indicating a detection result of at least one anatomical structure shown in captured image 32b of a subject is superimposed on captured image 32b. Viewer device 50a includes: display 54 that displays the medical image; and controller 53 that, when a size of an annotation indicating an anatomical structure having an abnormality (abnormal structure) among the at least one annotation is less than a threshold value, causes display 54 to superimpose probabilistic atlas 154a1 and the like indicating a normal structure assumed in a case where the abnormal structure is normal, on captured image 32b.

With this, even if the user could overlook the abnormality because the annotation indicating the abnormal structure is small, viewer device 50a display, for example, probabilistic atlas 154a1 indicating the normal structure assumed when this abnormal structure is normal. This can prevent the user from overlooking the abnormality of the anatomical structure. Hence, viewer device 50a can prevent a decrease in the diagnostic efficiency when the user makes a diagnosis using processed image 154a for instance.

Furthermore, probabilistic atlas 154a1 and the like may be displayed in a display form that is different from a display form of the at least one annotation.

With this, viewer device 50a can prevent the user from confusing between the annotation and probabilistic atlas 154a1 for instance. Hence, viewer device 50a can prevent a decrease in the diagnostic efficiency as compared to when the display form of the annotation is identical to that of probabilistic atlas 154a1 for instance.

Furthermore, the at least one annotation may have, for example, no light-transmittance, and probabilistic atlas 154a1 and the like may have for example, light-transmittance.

With this, viewer device 50a can prevent the user from confusing between the annotation and probabilistic atlas 154a1 for instance, simply by displaying probabilistic atlas 154a1 for instance with light-transmittance.

Furthermore, the at least one annotation may be, for example, displayed with no change in at least one of brightness or hue, and probabilistic atlas 154a1 and the like may be, for example, displayed by gradations where the at least one of brightness or hue gradually changes.

With this, viewer device 50a can prevent the user from confusing between the annotation and probabilistic atlas 154a1 for instance, simply by displaying probabilistic atlas 154a1 for instance by gradations.

Furthermore, when the annotation indicating the anatomical structure having the abnormality, probabilistic atlas 154a1, and the like are superimposed on captured image 32b, controller 53 may cause the annotation, probabilistic atlas 154a1, and the like to be displayed to make the annotation visible.

With this, even when displaying probabilistic atlas 154a1 for instance, viewer device 50a allows the user to visually identify the annotation detected by detector 42. More specifically, when making the diagnosis, the user can also use the annotation detected by detector 42.

Furthermore, controller 53 may determine whether the size of the annotation indicating the anatomical structure having the abnormality is less than the threshold value and, when determining that the size of the annotation is less than the threshold value, cause probabilistic atlas 154a1 and the like corresponding to the anatomical structure having the abnormality to be superimposed on captured image 32b.

With this, viewer device 50a can collectively perform the operations from the determination whether to superimpose the probabilistic atlas to the superimposition of the probabilistic atlas.

Furthermore, as described above, the medical image display method according to the present embodiments is a medical image display method of displaying a medical image. The medical image includes processed image 154a and the like in which at least one annotation indicating a detection result of at least one anatomical structure shown in captured image 32b of a subject is superimposed on captured image 32b. The medical image display method includes: when a size of an annotation indicating an anatomical structure having an abnormality among the at least one annotation is less than a threshold value (Yes at S110), superimposing probabilistic atlas 154a1 and the like indicating a normal structure assumed in a case where the anatomical structure having the abnormality is normal, on captured image 32b (S120). Furthermore, as described above, the program according to the present embodiments may cause a computer to execute the above-described medical image display method.

With this, the method achieves the same advantageous effects as viewer device 50a.

### (OTHER EMBODIMENTS)

Although the diagnostic support system, the medical image display system, and the medical image display method according to the present disclosure have been described based on the embodiments, the present disclosure is not limited to the embodiments. Those skilled in the art will readily appreciate that embodiments arrived at by making various modifications to the above embodiments or embodiments arrived at by selectively combining elements disclosed in the above embodiments without materially departing from the scope of the present disclosure may be included within one or more aspects of the present disclosure.

In the above embodiments, the viewer device is described as an example of the medical image display system. For example, the medical image display system may include at least one of the imaging device, the image server, and the abnormality detection system, in addition to the viewer device.

Furthermore, the above embodiments describe, as an example, that the detector performs detection using a neural network model. However, the learned machine learning model is not limited to a neural network model and may be any machine learning model. Moreover, the detection performed by the detector is not limited to the detection of the anatomical structure using the learned machine learning model. For example, the detector may detect an anatomical structure using the pattern matching technique. The method of detecting an anatomical structure from a captured image may use any known technique.

Furthermore, the above embodiments describe, as an example, that the segmentations are displayed in the two colors (yellow and blue) and in white (that is, displayed through brightness adjustment), with consideration for a user with color weakness. However, this is not intended to be limiting. For example, the viewer device may obtain, via the receiver, an input on whether the user who uses the viewer device has color weakness. Then, depending on the obtained input, the viewer device may change the display color of the annotation indicating the detected anatomical structure. For example, if the user has color weakness, the viewer device uses the display colors as described in the above embodiments. If the user has no color weakness, the viewer device may use three or more display colors. If the user has no color weakness, the viewer device may use a different display color for each of the anatomical structures, for example.

Furthermore, the medical image display method described in the above embodiments includes the processes performed by the viewer device. However, the processes are not limited to those described above. The method may further include at least one of the process performed by the abnormality detection system (the abnormality detection process, for example) or the process performed by the imaging device.

Furthermore, the above embodiments describe a segmentation as an example of the annotation. However, the annotation is not limited to this. For example, the annotation may be indicated by a frame that encloses an area detected by the detector with a line (a broken line, for example).

Furthermore, if each of the normal structure and the abnormal structure is displayed by a linear annotation, the width of the linear annotation indicating the reference structure in the above embodiments may be smaller than each of the widths of the linear annotations indicating the normal structure and the abnormal structure.

Furthermore, the above embodiments describe, as an example, that the processed image and the original image are displayed on one screen. However, this is not intended to be limiting. The processed image and the original image may be displayed alternately in response to a predetermined operation received.

Furthermore, the above embodiments describe, as an example, that the medical image displays two captured images. However, the number of captured images displayed on one medical image is not limited to two, and may be at least three. In this case, one of the at least three captured images indicates a result of detection performed by the detector of the abnormality detection system.

Furthermore, the functions of the abnormality detection system and the viewer device may be divided between the abnormality detection system and the viewer device in any way. For example, the image processor of the viewer device may be included in the abnormality detection system. Moreover, the abnormality detection system and the viewer device may be implemented by a single device or by a plurality of devices (at least three devices, for example). If the abnormality detection system and the viewer device are implemented by a plurality of devices, the structural components included in the abnormality detection system and the viewer device may be divided among the plurality of devices in any way. A communication method used among the plurality of devices is not limited to a particular method.

The dividing of the functional blocks in each of the block diagrams is one example. It is possible that a plurality of functional blocks are implemented into a single functional block, that a single functional block is divided into a plurality of functional blocks, and that a function executed by a functional block is partially executed by another functional block. Furthermore, similar functions of a plurality of functional blocks may be executed by a single hardware or software in parallel or by time division.

It should be noted that, in the above-described embodiments, each of the constituent elements may be implemented into a dedicated hardware or implemented by executing a software program suitable for the constituent element. Each of the constituent elements may be implemented when a program executing unit, such a central processing unit (CPU) or a processor, reads a software program from a recording medium, such as a hard disk or a semiconductor memory, and executes the readout software program. The processor is implemented by one or more electronic circuits including an integrated circuit (IC) or a Large Scale Integration (LSI). The electronic circuits may be integrated into a single chip or as a plurality of chips. The plurality of chips may be integrated into a single device or provided to a plurality of devices.

The order of processes described in the above embodiments is an example. The order of the processes may be changed, or at least a part of the processes may be performed in parallel.

An aspect of the present disclosure may be a computer program for causing a computer to execute the characteristic steps included in the medical image display method. An aspect of the present disclosure may be implemented to a non-transitory computer-readable recording medium, such as a Digital Versatile Disc (DVD), on which such a computer program is recorded. For example, such a program may be recorded on the recording medium and distributed. For example, it is possible that such a distributed program is installed in a device having another processor and executed by the other processor so as to allow the above-described steps.

The general or specific aspects of the present disclosure may be implemented to a system, a method, an integrated circuit, the above-described computer program, or the above-described non-transitory recording medium, or may be any combination of them. The program may be stored in the recording medium in advance, or provided to the recording medium via a wide area network such as the Internet.

### [Industrial Applicability]

The medical image display system and the medical image display method according to the present disclosure can be used as a system that detects the presence or absence of an abnormality in an anatomical structure included in a medical image, or more specifically, can be used as a system that assists a medical doctor in effectively making a diagnose.

### [Reference Signs List]

10 diagnostic support system
20 imaging device
30 image server
31a, 31b, 32a, 32b captured image
40 abnormality detection system
41, 51 communicator
42 detector
43 analyzer
50, 50a viewer device
52 receiver
53 controller
54 display
54a, 154a, 154b, 154c processed image
54b comparison image
54c structure label field
54c1 trachea shadow
54c2 measurement value
54d analysis result field
55 image processor
154a1, 154b1 probabilistic atlas
154b2, 154c1 segmentation
I1 initial state
I2a full-structure display
I2b, I3b original image
I3a selected-structure display

## Claims

1. A medical image display system that displays a medical image,
the medical image including:
a first display area for displaying a processed image in which at least one annotation indicating a result of detection of at least one anatomical structure shown in a captured image of a subject is superimposed on the captured image; and
a second display area for displaying at least one structure label for identifying the at least one anatomical structure,
the medical image display system comprising:
a display that displays the medical image; and
a controller that changes, in response to an input received for one of (i) the at least one annotation and (ii) the at least one structure label from a user, a display form of an other of (i) the at least one annotation and (ii) the at least one structure label.

2. The medical image display system according to claim 1, wherein
in response to the input of selection of a first annotation among the at least one annotation, the controller causes a measurement result of an anatomical structure corresponding to the first annotation to be additionally displayed in a structure label corresponding to the first annotation.

3. The medical image display system according to claim 1 or claim 2, wherein
in response to the input of selection of a first structure label among the at least one structure label, the controller causes the display to switch between displaying and not displaying an annotation corresponding to the first structure label.

4. The medical image display system according to any one of claims 1 to 3, wherein
the medical image further includes a third display area for displaying a comparison image to be compared with the processed image.

5. The medical image display system according to any one of claims 1 to 4, wherein
the medical image further includes a fourth display area for displaying a result of analysis performed on an abnormality in the at least one anatomical structure.

6. The medical image display system according to claim 5, wherein
in response to the input received for the one of (i) the at least one annotation and (ii) the at least one structure label, the controller causes the result of analysis performed on an anatomical structure identified from among the at least one anatomical structure based on the input to be displayed in the fourth display area.

7. The medical image display system according to any one of claims 1 to 6, wherein
the at least one annotation includes (i) a segmentation indicating an abnormal structure among the at least one anatomical structure and (ii) a segmentation indicating a normal structure among the at least one anatomical structure, and
the segmentation indicating the abnormal structure is light-transmissive.

8. The medical image display system according to claim 7, wherein
in response to the input received for one of the segmentation indicating the abnormal structure and a structure label corresponding to the segmentation, the controller causes a level of the light-transmittance of the segmentation to be changed.

9. The medical image display system according to any one of claims 1 to 6, wherein
the at least one annotation includes (i) a segmentation indicating an abnormal structure among the at least one anatomical structure and (ii) a segmentation indicating a normal structure among the at least one anatomical structure, and
the segmentation indicating the normal structure and a structure label corresponding to the segmentation are displayed in a first display color, and
the segmentation indicating the abnormal structure and a structure label corresponding to the segmentation are displayed in a second display color different from the first display color.

10. The medical image display system according to claim 9, wherein
in response to the input regarding one of (i) the segmentation indicating the abnormal structure and the segmentation indicating the normal structure and (ii) the at least one structure label, the controller causes at least one of the first display color or the second display color to be changed.

11. The medical image display system according to claim 9 or claim 10, wherein
the at least one annotation further includes a segmentation indicating a reference structure which is used as a structural reference of a corresponding one of the at least one anatomical structure in the processed image, and
the segmentation indicating the reference structure is displayed in an achromatic color.

12. The medical image display system according to any one of claims 1 to 11, wherein
the display form includes:
a first display mode in which each of the at least one annotation blinks; and
a second display mode in which a second annotation corresponding to the input among the at least one annotation blinks and an annotation other than the second annotation is not displayed, and
in response to the input of selection of the second annotation among the at least one annotation in the first display mode, the controller causes the first display mode to be switched to the second display mode.

13. A medical image display method of displaying a medical image,
the medical image including:
a first display area for displaying a processed image in which at least one annotation indicating a result of detection of at least one anatomical structure shown in a captured image of a subject is superimposed on the captured image; and
a second display area for displaying at least one structure label for identifying the at least one anatomical structure,
the medical image display method comprising
changing, in response to an input received for one of (i) the at least one annotation and (ii) the at least one structure label from a user, a display form of an other of (i) the at least one annotation and (ii) the at least one structure label.

14. A program for causing a computer to execute the medical image display method according to claim 13.
